# EUROPEAN PATENT SPECIFICATION

(11) **EP 0 370 974 B1**
(45) Date of publication and mention of the grant of the patent: **21.01.1998**
(21) Application number: 89830514.9
(22) Date of filing: 21.11.1989
(51) Int. Cl.: C07D 211/90, A61K 31/44

(54) **Process for preparing heterocyclic derivatives**
Verfahren zur Herstellung von heterocyclischen Derivaten
Procédé pour la préparation de dérivés hétérocycliques

(30) Priority: 21.11.1988 IT 2267788
(43) Date of publication of application: 30.05.1990
(73) Proprietor: GLAXO WELLCOME S.p.A., 37100 Verona (IT)
(72) Inventor: Pieraccioli, Daniele, Verona (IT)
(74) Representative: Aimi, Luciano

(56) References cited:
- FR-A- 2 569 402
- HOUBEN-WEYL: "Methoden der Organischen Chemie", vol. E5: "Carbonsäuren und Carbonsäure-Derivate", 1985, pages 141-146, Georg Thieme Verlag, Stuttgart, DE; G. SIMCHEN: "Orthocarbonsäure-Derivate"
- LIEBIGS ANNALEN CHEM., no. 5, 1974, pages 821-834; H. VORBRÜGGEN: "Reaktionen von Amidacetalen"

## Description

This invention relates to a novel process for preparing dihydropyridine derivatives useful in the treatment of cardiovascular disorders.

United Kingdom Published Specification GB 2164336A describes compounds of formula (I) 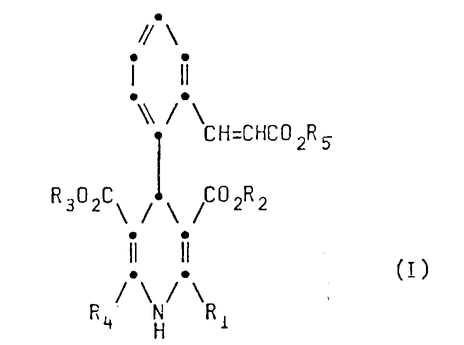 wherein
R₁ and R₄ independently represent C₁-₄ alkyl;
R₂ and R₃ independently represent a straight or branched C₁-₆ alkyl chain optionally interrupted by an oxygen atom; and R₅ represents a tertiary butyl group.

The group CH=CHCO₂R₅ in the compounds of formula (I) can exist in the cis (Z) or the trans (E) configuration and formula (I) is intended to refer to the (Z) end (E) forms individually as well as to mixtures of the two forms.

The compounds are useful for the treatment of cardiovascular disorders such as hypertension, angina pectoris, myocardial ischaemia, congestive heart failure, cerebral vascular and peripheral disorders.

A number of process for preparing the compounds of formula (I) are described in UK Specification GB 2164336A, including esterification of the corresponding acid of formula (I) in which R₅ represents a hydrogen. More particularly the specification teaches that the esterification may be effected by treating a compound of formula (I) in which R₅ is a hydrogen atom with an alkylating agent R₅X where R₅ is as defined in formula (I) and X is a leaving group such as a chloride, bromide, iodide or mesylate. Alternatively the specification teaches that the esterification may be carried out by converting the acid corresponding to the compound of formula (I) into an activated derivative thereof such as a mixed anhydride which is then reacted with an alcohol R₅OH or the corresponding alkoxide thereof in which R₅ is as defined in formula (I).

We have now found that the compounds of formula (I) may be advantageously prepared by reacting the corresponding acid of formula (II) with an appropriate amide acetal of the general formula RₐR_{b}NCR_{c}(OR₅)₂ (III) in which R₅ is as defined in formula (I) and Rₐ and R_{b} independently represent a C₁₋₄ alkyl group or together they form a stright alkylene chain containing 4 to 6 carbon atoms or the group (CH₂)₂O(CH₂)₂-; R_{c} represents a hydrogen atom, a C₁₋₃ alkyl group or Rₐ and R_{c} are linked together to form an alkylene chan containing 3 to 5 carbon atoms. This process has the particular advantage of giving the required ester in good yield.

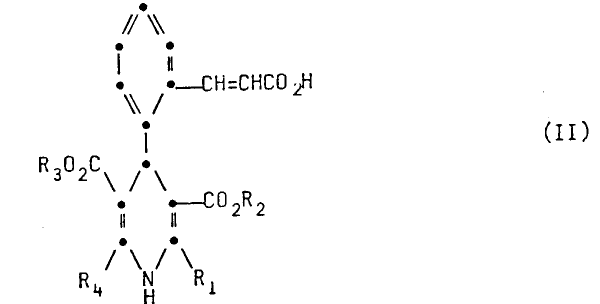

The invention therefore provides a process for the preparation of compounds of formula (I) in which the groups R₁, R₂, R₃, R₄ and R₅ have the meanings given above, which comprises esterification of an acid of formula (II) in which the groups R₁, R₂, R₃ and R₄ have the meanings defined above in formula (I) by reaction with said amide acetal capable of introducing the group R₅. Particularly preferred acetals are those in which Rₐ and R_{b} are C₁₋₃ alkyl, especially methyl and R_{c} represents hydrogen. The reaction is conveniently carried out in an asprotic solvent such as a hydrocarbon e.g. benzene or toluene, a halohydrocarbon such as methylene chloride, acetonitrile or dimethylformamide and preferably with heating e.g. 40-120°C.

The acetals of formula (III) are either known compounds or can be prepared by analogous methods to those described for the known compounds.

The process of the present invention is particularly useful for the preparation of compounds of formula (I) wherein R₁ and R₄ represents methyl, R₂ and R₃ independently represent C₁₋₄ alkyl e.g. methyl, ethyl, isopropyl or isobutyl or ethyl substituted by C₁₋₃ alkoxy e.g. methoxy or propoxy and R₅ represents a tertiary butyl group.

A specific compound which may be conveniently prepared by the processes of the present invention is (E)-4-(2-(3-(1,1-dimethylethoxy)-3-oxo-1-propenyl)phenyl)-1,4-dihydro-2,6-dimethyl-3,5-pyridinedi- carboxylic acid, diethyl ester.

The acids of formula (II) may be prepared by the process described in UK Specification GB 2164336A. More advantageously however the acids of formula (II) and more particularly the E isomers thereof, may be prepared in good yield from the reaction compound of formula (IV). 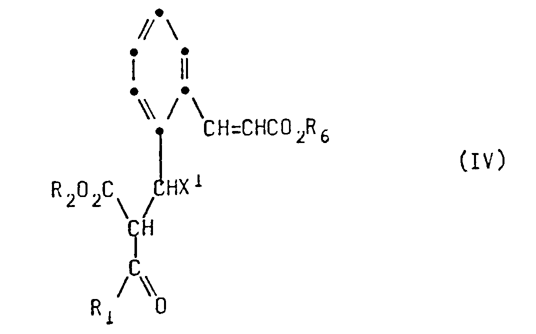 wherein R₁ and R₂ are as previously defined; X¹ represents a halogen atom e.g. chlorine, bromine or iodine and R₆ is a hydrogen atom or a carboxyl protecting group, with an amino ester (V), with subsequent deprotection as appropriate.

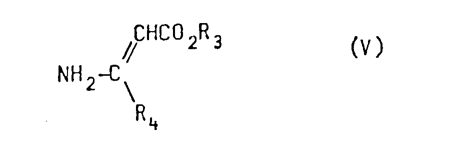

The reaction is conveniently carried out in a suitable solvent such as an alkanol e.g. ethanol or isopropanol, in the presence of a base, such as a trialkylamine e.g. triethylamine and preferably with heating e.g. 40-150°C.

If the carboxyl protecting group R₆ is not cleaved under the conditions of the reaction then this may be subsequently removed by conventional procedures. Examples of suitable protecting groups include C₁₋₆alkyl e.g. tertiary butyl, tetrahydropyranyl, tetrahydrofuranyl, trimethylsilyl, or activated methyl esters such as phthalimidomethyl, benzyloxymethyl or methoxyethoxymethyl groups.

Preferably the reaction is carried out using a haloketoester of formula (IV) where X represents chlorine and the group R⁶ represents a C₁₋₆ alkyl group, conveniently a tertiary butyl group.

The haloketoester (IV) may be prepared from the reaction of the aldehyde (VI) wherein R₆ is as defined above, with the ketoester (VII) wherein R₁ and R₂ are as defined above in the presence of the appropriate hydrogen halide (HX).

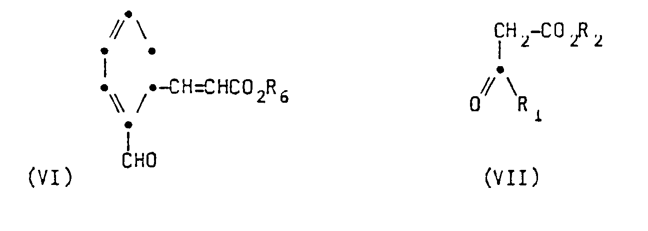

The reaction is conveniently carried out in a suitable non-aqueous solvent such as a hydrocarbon e.g. benzene, toluene or xylene. The reaction may be carried out at a temperature within the range -30° to 100°, more preferably between -10° to 30°C.

The aldehyde (VI) and the ketoesters (VII) are either known compounds or may be prepared by analogous methods to those described for the known compounds.
The following examples illustrate the invention. Throughout the examples reference to t.l.c. means thin layer chromatography on silica plates (Merck 60F-254).

### Intermediate 1

### (E)-2-(α-Chloro-2-(carboxyethenyl)phenylmethyl)-3-oxo-butanoic acid, ethyl ester

A mixture of 2-formylcinnamic acid (0.76g) and ethyl acetoacetate (0.56g) in toluene was treated with HCl gas, at 0°C, for 15 minutes, then was left standing with sealing at room temperature for 20 hrs. The solid was collected by filtration, washed quickly with toluene, then with petrol and dried in vacuo over P₂O₅. The title compound was obtained as a white solid (0.9g).
m.p. 171-173°C.
T.l.c. (methylene chloride/methanol, 9:1) Rf. = 0.53

### Intermediate 2

### (E)-4-(2-(2-Carboxyethenyl)phenyl)-1,4-dihydro-2,6-dimethyl-3,5-pyridinedicarboxylic acid, diethyl ester

A solution of Intermediate (1) (1.4g), ethyl 3-aminocrotonate (0.57g) and triethylamine (0.88g) in isopropanol (10ml) was refluxed for 18 hrs. The solvent was evaporated and the residue taken up with water and made alkaline with potassium bicarbonate. The mixture was washed with ethyl acetate, then treated with 10% HCl (pH 2-3) and extracted with ethyl acetate. The organic phase was washed with water, dried over MgSO₄ and the solvent evaporated to give a solid, which was recrystallised from petrol ether/ethyl acetate (1:1). The title compound was obtained as a white solid (1.4g).
m.p. 175-180°C (dec.)
T.l.c. (Methlene chloride/ethyl acetate/acetic acid, 8:2:1) (Rf = 0.4)

### Example 1

### (E)-4-(2-(3-(1,1-Dimethylethoxy)-3-oxo-1-propenyl)phenyl-1,4-dihydro-2,6-dimethyl-3,5-pyridinedicarboxylic acid diethyl ester

The compound of Intermediate 2 (1g) was suspended in toluene (10ml) and stirred at 80°C N,N-dimethylformamide di-tert-butyl acetal (4g) was added dropwise to the mixture at 80°C, in 15 minutes. The solution was stirred at 80°C for 4 hrs, then cooled, washed with water, sodium bicarbonate 5%, brine and dried over sodium sulphate. The solvent was evaporated and the residue (1.5g) was eluted on a silica gel column (ethyl acetate/cyclohexane, 7:3) to give the title compound as a white solid, (1g).
m.p. 173-175°C
T.l.c. (Methylene chloride/ethyl acetate, 9:1) Rf. = 0.40.

## Claims

1. A process for the preparation of compounds of formula (I) 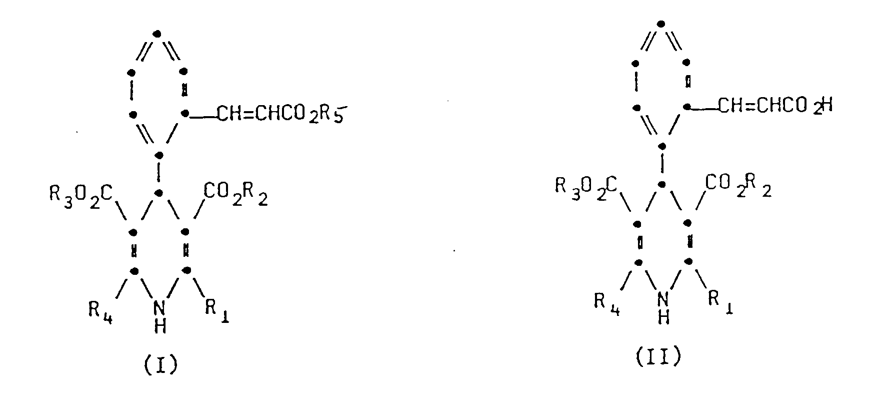 in which R₁ and R₄ each represent a methyl group, R₂ and R₃ each represent an ethyl group and R₅ represents a tertiary butyl group; which comprises esterification of an acid of formula (II) wherein the groups R₁, R₂, R₃ and R₄ have the meanings given above in formula (I) by reaction with an amide acetal of the general formula RₐR_{b}NCR₂(OR₅)₂ (III) in which R₅ is as defined in formula (I) and Rₐ and R_{b} independently represent a C₁₋₄alkyl group or together they form a straight alkylene chain containing 4 to 6 carbon atoms or the group -(CH₂)₂O(CH₂)₂-; R_{c} represents a hydrogen atom, a C₁₋₃alkyl group or Rₐ and R_{c} are linked together to form an alkylene chain containing 3 to 5 carbon atoms.

2. A process as claimed in claim 1 using an amide acetal of formula (III) in which the groups Rₐ and R_{b} independently represent a C₁₋₃alkyl group.

3. A process as claimed in claims 1 or 2 using an amide acetal of formula (III) in which the groups Rₐ and R_{b} each represent a methyl group.

4. A process as claimed in any of claims 1 to 3 using an amide acetal of formula (III) in which the group R_{c} represents a hydrogen atom.

5. A process as claimed in any of claims 1 to 4 in which the reaction is carried out in an aprotic solvent.

6. A process as claimed in any of claims 1 to 5 in which the reaction is carried out in a hydrocarbon solvent.

7. A process as claimed in any of claims 1 to 6 in which the reaction is carried out with heating.

8. A process as claimed in any of claims 1 to 7 for preparing the E-isomer of the compound of formula (I).

## Patentansprüche

1. Verfahren zur Herstellung von Verbindungen der Formel (I) 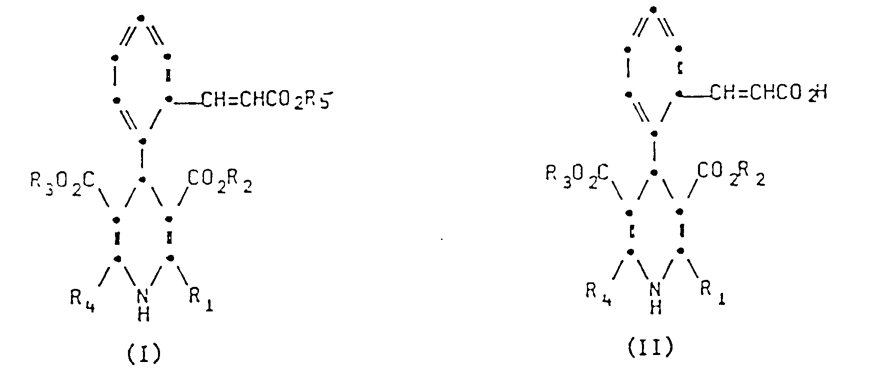 wobei R₁ und R₄ je eine Methylgruppe bedeuten, R₂ und R₃ je eine Ethylgruppe bedeuten und R₅ eine tertiäre Butylgruppe bedeutet; das die Veresterung einer Säure der Formel (II), wobei die Gruppen R₁, R₂, R₃ und R₄ dieselbe, oben für Formel (I) gegebene Bedeutung haben, durch Reaktion mit einem Amid-azetal der allgemeinen Formel RₐR_{b}NCR_{c}(OR₅)₂ (III) wobei R₅ wie in Formel (I) bestimmt ist und Rₐ und R_{b} unabhängig eine C₁₋₄Alkylgruppe bedeuten oder zusammen ein geradkettiges Alkylen mit 4 bis 6 Kohlenstoffatomen oder die Gruppe -(CH₂)₂O(CH₂)₂- bilden; R_{c} ein wasserstoffatom, eine C₁₋₃-Alkyilgruppe bedeutet oder Rₐ und R_{c} zusammen zu einer Alkylenkette mit 3 bis 5 Kohlenstoffatomen gebunden sind umfaßt.

2. Verfahren nach Anspruch 1, das ein Amido-azetal der Formel (III) wobei die Gruppen Rₐ und R_{b} unabhängig eine C₁₋₃-Alkylgruppe bedeuten benutzt.

3. Verfahren nach Ansprüchen 1 oder 2, das ein Amido-azetal der Formel (III) wobei die Gruppen Rₐ und R_{b} je eine Methylgruppe bedeuten benutzt.

4. Verfahren nach einem der Ansprüchen 1 bis 3, das ein Amido-azetal der Formel (III) wobei die Gruppe R_{c} ein Wasserstoffatom bedeutet benutzt.

5. Verfahren nach einem der Ansprüchen 1 bis 4, wobei die Reaktion in einem aprotischen Lösungsmittel durchgeführt wird.

6. Verfahren nach einem der Ansprüchen 1 bis 5, wobei die Reaktion in einem Kohlenwasserstofflösungsmittel durchgeführt wird.

7. Verfahren nach einem der Ansprüchen 1 bis 6, wobei die Reaktion unter Erwärmung durchgeführt wird.

8. Verfahren nach einem der Ansprüchen 1 bis 7 zur Herstellung des E-Isomeres der Verbindung der Formel (I).

## Revendications

1. Procédé de préparation de composés de formule (I) 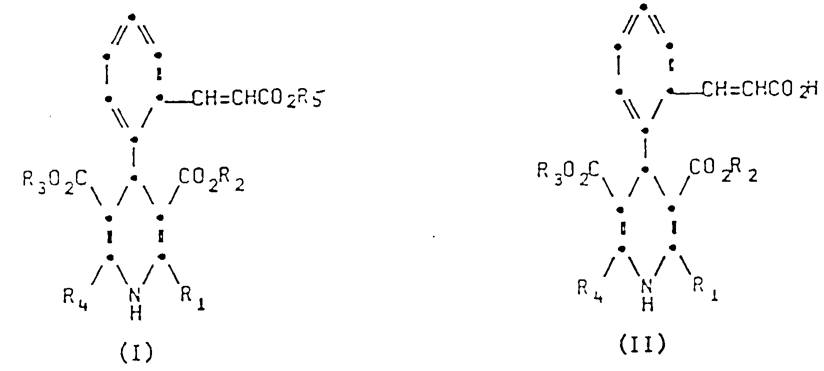 dans laquelle R₁ et R₄ représentent chacun un groupe méthyle, R₂ et R₃ représentent chacun un groupe éthyle et R₅ représente un groupe butyle tertiaire; procédé qui comprend l'estérification d'un acide de formule (II), où les groupes R₁, R₂, R₃, et R₄ ont les mêmes significations données dessus pour la formule (I), par réaction avec un amide-acétal de formule générale RₐR_{b}NCR_{c}(OR₅)₂ (III) dans laquelle R₅ est comme défini pour la formule (I) et Rₐ et R_{b} représentent indépendamment un groupe alkyle C₁₋₄ ou bien forment ensemble une chaîne alkylène linéaire contenant 4 à 6 atomes de carbone ou le groupe -(CH₂)₂O(CH₂)₂-, R_{c} représente un atome d'hydrogène, un groupe alkyle C₁₋₃ ou Rₐ et R_{c} sont liés ensemble à former une chaîne alkylène contenant 3 à 5 atomes de carbone.

2. Procédé selon la revendication 1, qui utilise un amide-acétal de formule (III) dans lequel les groupes Rₐ et R_{b} représentent indépendamment un groupe C₁₋₃ alkyle.

3. Procédé selon les revendications 1 ou 2, qui utilise un amide-acétal de formule (III) dans lequel les groupes Rₐ et R_{b} représentent chacun un groupe méthyle.

4. Procédé selon n'importe quelle des revendications 1 à 3, qui utilise un amide-acétal de formule (III) dans lequel le groupe R_{c} représente un atome d'hydrogène.

5. Procédé selon n'importe quelle des revendications 1 à 4, dans lequel la réaction est conduite dans un solvant aprotique.

6. Procédé selon n'importe quelle des revendications 1 à 5, dans lequel la réaction est conduite dans un solvant hydrocarbure.

7. Procédé selon n'importe quelle des revendications 1 à 6, dans lequel la réaction est conduite avec chauffage.

8. Procédé selon n'importe quelle des revendications 1 à 7, pour préparer l'isomère E du composé de formule (I).
